# NOUVEAU FASCICULE DE BREVET EUROPEEN

(11) **EP 0 884 998 B2**
(45) Date de publication et mention de la décision concernant l'opposition: **26.09.2007**
(45) Mention de la délivrance du brevet: 26.09.2001
(21) Numéro de dépôt: 97906244.5
(22) Date de dépôt: 21.02.1997
(51) Int. Cl.: A61K 8/46, A61Q 5/10

(54) **NOUVEAU PROCEDE POUR LA DEFORMATION PERMANENTE DES MATIERES KERATINIQUES**
NEUES VERFAHREN ZUR DAUERHAFTEN VERFORMUNG VON KERATINSUBSTANZEN
NOVEL METHOD FOR PERMING KERATINOUS MATERIALS

(30) Priorité: 26.02.1996 FR 9602355
(43) Date de publication de la demande: 23.12.1998
(73) Titulaire: L'ORÉAL, 75008 Paris (FR)
(72) Inventeur: DE LABBEY, Arnaud, F-93600 Aulnay-sous-Bois (FR); NGUYEN, Lylan, F-94240 L'Hay-les-Roses (FR)
(74) Mandataire: Dossmann, Gérard
(86) Numéro de dépôt international: PCT/FR1997/000327
(87) Numéro de publication internationale: WO 1997/030683

(56) Documents cités:
- EP-A- 0 344 653
- EP-A- 0 514 282
- EP-A- 0 577 473
- DE-A- 3 707 415
- DE-A- 4 119 044
- FR-A- 1 455 788
- NL-A- 6 514 890
- US-A- 2 719 813
- US-A- 2 719 814
- US-A- 2 719 815
- US-A- 2 869 559
- US-A- 3 099 603
- US-A- 3 840 656
- US-A- 4 273 143
- K.Schrader:Grundlagen und Rezepturen der Kosmetika,2ème édition, Hüthig-Verlag Heidelberg(1989),p.823-840
- Charles Zviak:The Science of Hair Care, Marcel Dekker, New York 1986 ,p.183-212:Permanent Waving and Hair Straightening.
- J.Soc.Cosmet.Chem.,38,99-107(mars/avril 1987);Reduction of hair in the presence of exogenous disulfide
- J.St.Jellinek:Kosmetologie,Hüthig-Verlag Heidelberg,3ème édition 1976,p-680-701
- Cosmetics and Toiletries,109,69-78(mars 1994):Cysteine Waving Lotions
- Hugo Janistyn:Taschenbuch der modernen Parfümerie und Kosmetik, 4ème édition 1974,Wissenschaftliche Verlagsgesellschaft Stuttgart, p.421-430

## Description

La présente invention concerne un procédé de traitement des fibres kératiniques, en particulier des cheveux, en vue d'obtenir une déformation permanente de ces dernières, en particulier sous la forme de cheveux permanentés.

On sait que la technique la plus usuelle pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres) une composition contenant un agent réducteur (étape de réduction) puis, après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'un tel procédé classique de déformation permanente des cheveux (procédé en deux temps) contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique, l'acide thioglycolique ainsi que ses esters, notamment le monothioglycolate de glycérol.

A cet égard, et bien que possédant une odeur désagréable, l'acide thioglycolique est particulièrement efficace, et constitue ainsi le composé de référence en permanente pour réduire les liaisons disulfures de la kératine.

De façon habituelle, la matière kératinique est enroulée sur des rouleaux et la composition réductrice est appliquée sur cette dernière durant un temps pouvant aller de 2 à 40 minutes afin de laisser au réducteur le temps d'agir sur les cheveux. La matière kératinique est ensuite abondamment rincée.

L'étape suivante constitue le deuxième temps d'un procédé classique de déformation permanente d'une matière kératinique et consiste à appliquer sur cette dernière une composition oxydante ou fixateur afin de « fixer » la matière kératinique ou les cheveux selon la forme désirée.

En ce qui concerne les compositions oxydantes nécessaires à la mise en oeuvre de cette étape de fixation, on fait le plus souvent appel, dans la pratique, à des compositions à base d'eau oxygénée ou de bromates alcalins. Ces compositions sont appliquées sur la matière kératinique enroulée sur les rouleaux durant un temps relativement long (fixation) pouvant aller jusqu'à 30 minutes. Elles sont généralement sous la forme de lotions très liquides dont il est difficile de contenir l'application à la seule fibre kératinique. Il arrive ainsi qu'un excès de ces compositions coule par exemple le long du cou de la cliente rendant cette étape particulièrement désagréable pour cette dernière. Après cette opération, la fibre kératinique est une dernière fois abondamment rincée.

Un tel procédé permet certes d'obtenir des cheveux permanentés présentant une belle frisure mais il présente un certain nombre d'inconvénients : il est long et fastidieux pour celui qui le met en oeuvre et très inconfortable pour celui ou celle qui le subit.

Afin de remédier à certains de ces désagréments, et dans le but de simplifier les opérations de permanentes en général, on a développé de nouveaux procédés de permanente qu'on appelle permanentes « auto-régulées ». Dans ces procédés, la composition réductrice comprend généralement, en plus de l'agent réducteur classique semblable à celui utilisé dans un procédé classique de déformation permanente (sulfites, thiols ...), un disulfure qui est en excès par rapport à l'agent réducteur classique et dont le rôle est d'oxyder les restes -SH libérés par la réduction de la cystine et de reconstituer ainsi instantanément les liaisons cystines. L'avantage de ces procédés est de rendre facultative, et de préférence inutile (permanente auto-neutralisante), l'étape de fixation et donc l'application avec temps de pause de la composition oxydante. Ces permanentes sont réalisées en un seul temps. Il s'ensuit un meilleur confort pour la cliente (le procédé est beaucoup plus rapide) ainsi qu'une facilité d'utilisation pour le coiffeur.

Cependant, on remarque que la frisure obtenue par de tels procédés est très souvent insuffisante et qu'elle se dégrade très rapidement dans le temps. De plus, l'étape de fixation étant inexistante, il subsiste longtemps après et même après un shampooing, une odeur désagréable due à l'excès de cheveux réduits.

La présente invention a notamment pour but de résoudre les problèmes ci-dessus. Plus précisément, elle a pour but de proposer un nouveau procédé de traitement de déformation permanente des fibres kératiniques qui soit plus simple, plus rapide et plus confortable pour la cliente qu'un procédé classique en deux temps et également plus efficace en terme de frisure qu'un procédé en un seul temps.

Or, la demanderesse a maintenant constaté que si l'on utilise une composition réductrice comprenant une association disulfure/thiol dans une gamme de rapports donnés, on peut simplifier significativement l'étape de fixation d'un procédé classique de déformation permanente des cheveux tout en obtenant une frisure belle et résistante.

Ainsi, la présente invention a pour objet un nouveau procédé de traitement des fibres kératiniques, en particulier des cheveux, en vue d'obtenir une déformation permanente de ces dernières, en particulier sous le forme de cheveux permanentes, ledit procédé étant **caractérisé par** le fait qu'il comprend les étapes suivantes (i) on applique sur les fibres kératiniques à traiter une composition réductrice comprenant une association disulfure/thiol, le rapport pondéral disulfure/thiol étant compris entre 0,1 et 1, les moyens, c'est-à-dire des rouleaux, nécessaires à la mise sous tension mécanique des fibres kératiniques étant mis en oeuvre avant ou pendant la dite application, (ii) on rince les fibres kératiniques, (iii) puis on sépare les moyens de mise sous tension utilisés à l'étape (i) des fibres kératiniques par découlage des fibres kératiniques entre la phase de réduction et la phase de fixation, (iv) on réalise une légère oxydation des fibres kératiniques consistant en l'application d'une composition oxydante avec un temps de pause inférieur à 2 minutes sur les fibres kératiniques, (v) et éventuellement on rince à nouveau les fibres kératiniques.

Un tel procédé est particulièrement simple et rapide. En effet, le choix spécifique du rapport pondéral disulfure/thiol dans la composition réductrice rend possible, lors de l'étape de fixation, une oxydation très légère. Ainsi, la composition oxydante peut être appliquée sur les fibres kératiniques avec un très faible temps de pause, voire sans temps de pause du tout et/ou la concentration en oxydant dans la composition oxydante peut être faible. Cette composition peut ainsi se présenter sous différentes formes telles que mousse, gel, lotion épaissie qui sont particulièrement simples et rapides à utiliser.

Une telle oxydation légère présente l'avantage de limiter la dégradation mécanique du cheveu que l'on constate habituellement lors de l'étape de fixation d'un procédé classique en deux temps avec un long temps de pause de la composition oxydante.

Le rapport pondéral disulfure/thiol particulier autorise également le déroulage des fibres kératiniques, et en particulier des cheveux, avant l'étape de fixation, et ceci sans risque aucun d'abîmer le cheveu ou la frisure. Le procédé de permanente est ainsi considérablement simplifié.

Un autre avantage d'un tel procédé est qu'il permet d'obtenir des cheveux permanentés présentant une frisure particulièrement belle et résistante dans le temps.

D'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute fibre kératinique en général, notamment cils, moustaches, poils, laine et autres.

La composition réductrice utilisée dans le procédé selon l'invention contient au moins un agent actif approprié pour la réduction des liaisons disulfures de la kératine sous la forme d'un thiol. Les thiols préférentiellement utilisés selon la présente invention sont choisis parmi l'acide thioglycolique et ses sels, le monothioglycolate de glycérol ou de glycol, la cystéamine et ses dérivés acylés en C₁-C₄ tels que la N-acétyl cystéamine ou la N-propionyl cystéamine, la cystéine, la N-acétyicystéine, les esters de cystéine, tels que le cystéïnate de glycérol, les N-mercaptoalkylamides de sucres tels que le N-(mercapto-2-éthyl)gluconamide, l'acide thiolactique et ses esters tels que le monothiolactate de glycérol, l'acide 3-mercaptopropionique et ses esters, tels que le 3-mercaptopropionate de glycérol, l'acide thiomalique, l'acide 2-hydroxy 3-mercaptopropionique et ses esters, tels que le 2-hydroxy 3-mercaptopropionate de glycérol, la panthétéine, le thioglycérol, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000, les dérivés des acides N-(mercaptoalkyl) succinamiques ou des N-(mercaptoalkyl) succiminides décrits dans la demande de brevet EP-A-465 342, les alkylaminomercaptoalkylamides décrits dans la demande de brevet EP-A-514 282, le mélange de thioglycolate d'hydroxy-2-propyle et de thioglycolate d'hydroxy-2-méthyl-1 éthyle décrit dans la demande de brevet FR-A-2 679 448, les N-mercaptoalkyl alcanediamides décrits dans la demande de brevet EP-A-653 202.

On utilise de préférence l'acide thioglycolique, l'acide thiolactique, la cystéïne et ses divers dérivés, la cystéamine et ses dérivés, l'acide 3-mercaptopropionique, ainsi que leurs esters ou leurs sels, notamment le monothioglycolate de glycérol.

Ces agents actifs peuvent être utilisés seuls ou en mélange.

Lorsque l'on utilise l'acide thioglycolique, l'acide thiolactique, l'acide 3-mercaptopropionique, l'acide 2-hydroxy 3-mercaptopropionique, la cystéine ou la cystéamine, ou l'un de leurs sels ou de leurs dérivés, à titre d'agent réducteur, le pH de l'ensemble de la composition selon l'invention est de préférence compris entre 6 et 11,5 et encore plus préférentiellement entre 7 et 10.

Lorsque l'on utilise les esters de l'acide thioglycolique ou de l'acide thiolactique ou de l'acide 3-mercaptopropionique, de la cystéïne ou de l'acide 2-hydroxy 3-mercaptopropionique, à titre d'agent réducteur, le pH de l'ensemble de la composition selon l'invention est de préférence compris entre 5 et 10 et encore plus préférentiellement entre 6 et 9.

Selon une caractéristique essentielle de la présente invention, les compositions réductrices utilisées contiennent également au moins un deuxième agent actif sous la forme d'un disulfure. Parmi les disulfures connus, on peut notamment mentionner l'acide dithiodiglycolique, le dithioglycérol, l'acide dithiodilactique, le disulfure de l'acide 3-mercaptopropionique, la cystamine, la N, N'-diacétyl-cystamine, la cystine, la pantéthine, et les disulfures des N-(mercaptoalkyl) ω-hydroxyalkyl-amides décrits dans la demande de brevet EP-A-354 835, les disulfures des N-mono ou N,N-dialkylmercapto-4 butyramides décrits dans la demande de brevet EP-A-368 763, les disulfures des aminomercapto-alkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A-465 342, les disulfures des alkylaminomercaptoalkylamides décrits dans la demande de brevet EP-A-514 282 et les disulfures des N-mercaptoalkyl alcane diamides décrits dans la demande de brevet EP-A-653 202.

De préférence, les compositions réductrices conformes à la présente invention comprennent un thiol en association avec son disulfure correspondant. Les associations disulfure/thiol correspondant préférées selon la présente invention sont choisies parmi les associations suivantes : acide dithiodiglycolique ou ses sels/acide thioglycolique ou ses sels, acide dithiodilactique ou ses sels/acide thiolactique ou ses sels, cystine/cystéine, cystamine/cystéamine, disulfure de l'acide 3-mercaptopropionique/ acide 3-mercaptopropionique.

Les thiols mentionnés ci-avant sont généralement présents à une concentration qui peut être comprise entre 1 et 20 %, de préférence entre 5 et 15 %, en poids par rapport au poids total de la compositon réductrice.

La concentration en disulfure est déterminée en fonction de la concentration en thiol de telle sorte que le rapport pondéral disulfure/thiol soit compris entre 0,1 et 1, de préférence entre 0,3 et 0,8. Ainsi, la concentration en disulfure dans les compositions réductrices du procédé selon l'invention est généralement comprise entre 0,1 et 20 % en poids par rapport au poids total de la composition.

Les pH des compositions réductrices peuvent être ajustés classiquement par ajout d'agents basifiants, tels que par exemple la soude, l'ammoniaque, la monoéthanolamine, la diéthanolamine, la triéthanolamine, l'isopropanolamine, les dérivés de diaminopropane tels que le 1,3-diaminopropane, le 2-amino-2-méthyl-1-propanol, le 2-amino-2-méthyl-1,2-propanediol, un carbonate ou bicarbonate de métal alcalin ou d'ammonium, un carbonate ou bicarbonate d'amines primaires, secondaires ou tertiaires, ou un carbonate organique tel que le carbonate de guanidine, tous ces composés pouvant bien entendu être pris seuls ou en mélange.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol, du glycérol, du propylène glycol ou encore du diéthylène glycol à une concentration maximale de 20 % par rapport au poids total de la composition.

La composition réductrice peut également contenir des additifs cosmétiques bien connus pour ce type de composition tels que des polymères cationiques, des surfactants, des parfums ou encore des silicones.

L'application de la composition réductrice peut être réalisée avant, pendant ou après l'habituelle étape de mise sous tension des cheveux. De préférence, cette application est réalisée après l'étape de mise sous tension des cheveux.

Avant de procéder à l'étape suivante de rinçage, il convient, de manière classique, de laisser reposer pendant quelques minutes, généralement entre 2 et 40 minutes, de préférence entre 5 et 20 minutes, la chevelure sur laquelle a été appliquée la composition réductrice, et ceci de façon à bien laisser le temps aux actifs d'agir correctement sur les cheveux. Cette phase d'attente est effectuée généralement en laissant reposer à l'air libre (température ambiante) la chevelure traitée, mais elle peut être également effectuée à une température plus élevée. Pendant cette phase d'attente, on prend soin que les cheveux ne sèchent pas complètement et restent humides jusqu'au moment de la mise en oeuvre de l'étape suivante (à cet effet, utilisation possible de bonnets, de gels de protection par exemple).

Dans la deuxième étape du procédé (étape (ii)), les cheveux imprégnés de la composition réductrice sont rincés soigneusement.

Selon une caractéristique essentielle du procédé selon l'invention, la troisième étape (étape (iii)) consiste à séparer les moyens, c'est-à-dire des rouleaux, utilisés pour la mise sous tension mécanique des fibres kératiniques de ces dernières avant l'application d'une composition oxydante. On procède ainsi au déroulage des fibres kératiniques, et en particulier des cheveux, entre la phase de réduction et la phase de fixation.

Grâce au rapport spécifique choisi (poids en disulfure)/(poids en thiol) dans la composition réductrice, cette étape de déroulage des cheveux ainsi que l'étape suivante (étape (iv) de fixation) ne présentent aucun risque de cassure des fibres kératiniques.

Une conséquence directe de ce déroulage précoce par rapport à un procédé classique de permanente est un confort amélioré pour la cliente : en effet, les cheveux ne restent sous tension que durant le premier temps du procédé. La mise en oeuvre de la suite du procédé de déformation permanente est également grandement facilitée pour le coiffeur comme on peut le constater dans l'étape suivante.

L'étape suivante (étape (iv)) consiste à réaliser sur la chevelure déroulée une légère oxydation. Au sens de la présente invention, on entend, par réalisation d'une légère oxydation, l'application d'une composition oxydante dont la concentration en oxydant est faible et/ou l'application d'une composition oxydante avec faible, voire sans, temps de pause.

De préférence, l'oxydation légère est due à un faible temps de pause de la composition oxydante. En effet, comme il a été expliqué ci-dessus, la composition réductrice particulière à l'invention (rapport pondéral disulfure/thiol dans une fourchette spécifique) permet de réaliser, lors de l'étape de fixation, une oxydation beaucoup plus faible que lors d'un procédé classique de permanente.

En conséquence, selon la présente invention, la compositon oxydante nécessaire à cette oxydation est appliquée sur les fibres kératiniques avec un temps de pause inférieur à 2 minutes, de préférence inférieur à 60 secondes.

Cette composition légèrement oxydante comprend un agent oxydant tel que l'eau oxygénée ou encore des bromates alcalins. La concentration en eau oxygénée peut varier de 1 à 9 volumes, de préférence de 1 à 8 volumes. La concentration en bromates peut généralement varier de 1 à 7 %.

Selon un mode préféré de réalisation de l'invention, la composition oxydante a une concentration en eau oxygénée de 5 à 8 volumes et est appliquée sur la fibre kératinique durant un temps inférieur à 60 secondes.

Selon un autre mode préféré de réalisation de l'invention, la composition oxydante a une concentration en eau oxygénée de 2 à 5 volumes et est appliquée sur la fibre kératinique durant un temps compris entre 1 et 2 minutes.

Le véhicule des compositions réductrice et oxydante utilisées selon l'invention est de préférence l'eau ou une solution hydroalcoolique d'un polyol ou d'un alcool inférieur tel que l'éthanol, l'isopropanol, le tertiobutanol ou le propylène glycol.

L'eau oxygénée peut être stabilisée par exemple par la phénacétine, l'acétanilide, les phosphates mono et trisodiques ou par le sulfate d'hydroxy-8 quinoléine.

La composition oxydante peut également contenir des agents alcalinisants ou acidifiants, des agents conservateurs, des agents séquestrants, des opacifiants, des agents épaississants ou encore des additifs cosmétiques bien connus pour ce type de composition tels que des stabilisants, des polymères cationiques, des tensioactifs, des agents de viscosité, des silicones ou encore des parfums.

Cette composition légèrement oxydante peut se présenter sous différentes formes : shampooing, gel, mousse, crème, lotion épaissie...Ces diverses formes sont rendues possibles par le fait, d'une part, que cette composition est peu oxydante, et d'autre part, qu'elle est appliquée sur des fibres kératiniques ou des cheveux libres. Ainsi, elle peut être appliquée très simplement, de la même manière qu'un soin habituel, tel qu'un shampooing ou un gel, que l'on fait légèrement mousser en massant les cheveux très légèrement, puis que l'on élimine en rinçant les fibres kératiniques avec de l'eau.

Il est clair ici que le procédé selon l'invention permet de résoudre les problèmes d'inconfort rencontrés (longs temps de pause, composition oxydante coulant désagréablement sur la peau) lors des procédés classiques de déformation permanente des cheveux.

Enfin, la dernière étape (étape (v)) de ce procédé est une étape classique de rinçage des fibres kératiniques ainsi traitées.

On obtient une belle frisure régulière de la racine à la pointe, qui présente une bonne tenue même après plusieurs shampooings.

Des exemples concrets, destinés à illustrer l'invention sans la limiter, vont maintenant être donnés.

Dans ce qui suit et ce qui précède, les quantités sont exprimées en pourcentage en poids par rapport au poids total des compositions.

### EXEMPLE 1 :

Dans cet exemple, on a comparé deux procédés de déformation permanente des cheveux : un procédé A selon l'invention et un procédé B représentatif d'un procédé de permanente auto-neutralisante connu. Un tel procédé de permanente auto-neutralisante nécessite une composition réductrice comprenant un taux élevé de disulfure et n'utilise pas de soin terminal oxydant.

### Procédé A (invention) :

On prépare selon l'invention une composition réductrice de déformation permanente des cheveux en procédant au mélange des matières premières suivantes :
- acide thioglycolique 9,4 %
- séquestrant 0,4 %
- ammoniaque (à 20% MA) 11,5 %
- carbonate d'ammonium 5,8 %
- dithioglycolate d'ammonium (40 % MA) 15 %
- tensio-actif 0,5 % MA
- parfum 0,5 %
- peptisant 1 %
- eau déminéralisée qsp 100 %
pH = 8,4.

Le rapport (poids de disulfure)/(poids de thiol) de cette composition est ainsi d'environ 0,64.

Cette composition est appliquée sur les cheveux naturels mouillés et préalablement enroulés sur des bigoudis de permanente. Après avoir laissé agir la composition pendant 15 minutes, on rince abondamment à l'eau puis on enlève les bigoudis.

On applique alors le soin terminal de composition suivante :
- eau oxygénée (200 vol) 4,8 %
- tensio-actif 0,5 % MA
- hydroxyéthylcellulose 1 %
- peptisant 0,5 %
- parfum 0,3 %
- acide citrique qsp pH=3
- eau déminéralisée qsp 100 %

Ce soin a une texture épaissie : il ne coule pas, d'où un confort amélioré pour la cliente et une application facilitée. Ainsi, on applique ce soin comme un shampooing qu'on fait légèrement mousser dans les cheveux sans temps de pause. On rince tout de suite la chevelure.

On obtient alors une belle frisure régulière de la racine à la pointe. Les cheveux ne présentent aucune odeur résiduelle. De plus cette frisure est résistante dans le temps : elle a ainsi une tenue de 8 semaines.

### Procédé B : (comparatif)

On prépare une composition réductrice convenant pour une déformation permanente auto-neutralisante des cheveux en procédant au mélange des fibres premières suivantes :
- acide thioglycolique 6 %
- séquestrant 0,4 %
- ammoniaque (à 20% MA) 8,3 %
- carbonate d'ammonium 5,8 %
- dithioglycolate d'ammonium (40 % MA) 20 %
- tensio-actif 0,5 % MA
- parfum 0,5 %
- peptisant 1 %
- eau déminéralisée qsp 100%
pH = 8,4

Le rapport (poids de disulfure)/(poids de thiol) de cette composition est ainsi d'environ 1,33.

Cette composition est appliquée sur des cheveux naturels mouillés préalablement enroulés sur des bigoudis de permanente. Après avoir laissé agir la composition pendant 15 minutes, on rince abondamment à l'eau puis on enlève les bigoudis.

La frisure obtenue avec un tel procédé est très faible. De plus, malgré un rinçage très prolongé après l'application de la composition réductrice, une désagréable odeur de cheveux réduits persiste dans les cheveux. La frisure disparaît dès le premier shampooing alors que l'odeur résiduelle désagréable est toujours présente.

### EXEMPLE 2 :

On applique le procédé A de l'exemple 1 sur des cheveux moyennement sensiblisés par un traitement de décoloration, avec les compositions réductrice et oxydante suivante :

### Composition réductrice :

- acide thioglycolique 7 %
- séquestrant 0,2 %
- ammoniaque (à 20% MA) 6,5 %
- carbonate d'ammonium 5,8 %
- dithioglycolate d'ammonium (40 % MA) 10 %
- tensio-actif 0,5 % MA
- parfum 0,5 %
- peptisant 1 %
- eau déminéralisée qsp 100 %
pH = 8.

Le rapport (poids de disulfure)/(poids de thiol) de cette composition est ainsi d'environ 0,57.

### Lotion mousse terminale (composition légèrement oxydante) :

- eau oxygénée (200 vol) 1,2 % (2 vol)
- tensio-actif 1 % MA
- acide citrique qsp pH=3
- eau déminéralisée qsp 100 %

La frisure obtenue selon ce procédé est belle et régulière de la racine à la pointe. Elle a une très bonne tenue dans le temps.

### EXEMPLE 3 :

Dans cet exemple, on a comparé deux procédés de déformation permanente des cheveux : un procédé A selon l'invention et un procédé B utilisant des compositions réductrice et oxydante d'un procédé classique de permanente en deux temps mais pour lequel on a procédé au déroulage des cheveux avant l'étape de fixation.

Le procédé A est le même que celui réalisé dans l'exemple 1 avec les mêmes compositions réductrice et oxydante que dans l'exemple 1.

### Procédé B : (comparatif)

On prépare une composition réductrice convenant pour une déformation permanente classique des cheveux en procédant au mélange des matières premières suivantes :
- acide thioglycolique 9,4 %
- séquestrant 0,4 %
- ammoniaque (à 20% MA) 11,5 %
- carbonate d'ammonium 5,8 %
- tensio-actif 0,5 % MA
- parfum 0,5 %
- peptisant 1 %
- eau déminéralisée qsp 100 %
pH = 8,4

Cette composition est appliquée sur des cheveux naturels mouillés et préalablement enroulés sur des bigoudis de permanente. Après avoir laissé agir la composition pendant 15 minutes, on rince abondamment à l'eau puis on enlève les bigoudis.

On applique alors la composition oxydante (fixateur) classique suivante:
- eau oxygénée 8 vol
- acide citrique qs
- eau déminéralisée qsp 100 %
pH = 3

La frisure obtenue est très insatisfaisante en comparaison avec la belle frisure régulière et résistante obtenue avec le procédé A dans l'exemple 1.

## Revendications

1. Procédé de traitement des fibres kératiniques, en particulier des cheveux, en vue d'obtenir une déformation permanente de ces dernières, en particulier sous la forme de cheveux permanentés, ledit procédé étant **caractérisé par le fait qu'**il comprend les étapes suivantes : (i) on applique sur les fibres kératiniques à traiter une composition réductrice comprenant une association disulfure/thiol, le rapport pondéral disulfure/thiol étant compris entre 0,1 et 1, les moyens, c'est-à-dire des rouleaux, nécessaires à la mise sous tension mécanique des fibres kératiniques étant mis en oeuvre avant ou pendant ladite application, (ii) on rince les fibres kératiniques, (iii) puis on sépare les moyens de mise sous tension utilisés à l'étape (i) des fibres kératiniques par découlage des fibres kératiniques entre la phase de réduction et la phase de fixation, (iv) on réalise une légère oxydation des fibres kératiniques, consistant en l'application d'une composition oxydante avec un temps de pause inférieur à 2 minutes sur les fibres kératiniques, (v) et éventuellement on rince à nouveau les fibres kératiniques.

2. Procédé selon la revendication 1, **caractérisé par le fait que** le rapport pondéral disulfure/thiol est compris entre 0,3 et 0,8.

3. Procédé selon la revendication 1 ou 2, **caractérisé par le fait que** l'association disulfure/thiol est choisie parmi les associations suivantes : acide dithiodiglycolique ou ses sels/acide thioglycolique ou ses sels, acide dithiodilactique ou ses sels/acide thiolactique ou ses sels, cystine/cystéine, cystamine/cystéamine, disulfure de l'acide mercaptopropionique/ acide mercaptopropionique.

4. Procédé selon l'une quelconque des revendications précédentes, **caractérisé par le fait que** le thiol est présent dans la composition réductrice à une teneur allant de 1 à 20 % en poids, par rapport au poids total de ladite composition.

5. Procédé selon la revendication 4, **caractérisé par le fait que** ladite teneur va de 5 à 15 %.

6. Procédé selon la revendication 1, **caractérisé par le fait que** ledit temps de pause est inférieur à 60 secondes.

7. Procédé selon l'une quelconque des revendications 1 à 6, **caractérisé par le fait que** la composition oxydante comprend de l'eau oxygénée.

8. Procédé selon la revendication 7, **caractérisé par le fait que** la concentration en eau oxygénée dans la composition oxydante va de 1 à 9 volumes.

9. Procédé selon la revendication 8, **caractérisé par le fait que** ladite concentration va de 1 à 8 volumes.

10. Procédé selon l'une quelconque des revendications 1 à 9 **caractérisé par le fait que** la composition oxydante se présente sous la forme d'un shampooing, d'un gel, d'une mousse, d'une lotion épaissie ou encore d'une crème.

## Claims

1. Process for treating keratin fibres, in particular the hair, in order to obtain a permanent reshaping of this hair, in particular in the form of permanent-waved hair, the said process being **characterized in that** it comprises the following steps: (i) a reducing composition comprising a disulphide/thiol combination is applied to the keratin fibres to be treated, the disulphide/thiol weight ratio being between 0.1 and 1, the means, i.e. rollers, required to place the keratin fibres under mechanical tension being used before or during the said application, (ii) the keratin fibres are rinsed, (iii) the means for placing the keratin fibres under tension used in step (i) are then removed by unrolling of the keratin fibres between the reducing phase and the fixing phase, (iv) the keratin fibres are mildly oxidized, which consists in applying an oxidizing composition with a standing time of less than 2 minutes on the keratin fibres, and (v) the keratin fibres are optionally rinsed again.

2. Process according to Claim 1, **characterized in that** the disulphide/thiol weight ratio is between 0.3 and 0.8.

3. Process according to Claim 1 or 2, **characterized in that** the disulphide/thiol combination is chosen from the following combinations: dithiodiglycolic acid or its salts/thioglycolic acid or its salts, dithiodilactic acid or its salts/thiolactic acid or its salts, cystine/cysteine, cystamine/cysteamine, mercaptopropionic acid disulphide/ mercaptopropionic acid.

4. Process according to any one of the preceding claims, **characterized in that** the thiol is present in the reducing composition at a content ranging from 1% to 20% by weight relative to the total weight of the said composition.

5. Process according to Claim 4, **characterized in that** the said content ranges from 5% to 15%.

6. Process according to Claim 1, **characterized in that** the said standing time is less than 60 seconds.

7. Process according to any one of Claims 1 to 6, **characterized in that** the oxidizing composition comprises aqueous hydrogen peroxide solution.

8. Process according to Claim 7, **characterized in that** the concentration of aqueous hydrogen peroxide solution in the oxidizing composition ranges from 1 to 9 volumes.

9. Process according to Claim 8, **characterized in that** the said concentration ranges from 1 to 8 volumes.

10. Process according to any one of Claims 1 to 9, **characterized in that** the oxidizing composition is in the form of a shampoo, a gel, a mousse, a thickened lotion or a cream.

## Patentansprüche

1. Verfahren zur Behandlung von Keratinfasern und insbesondere zur Behandlung des Haares, um eine dauerhafte Verformung des Haares, insbesondere in Form von dauergewelltem Haar, zu erzielen, wobei das Verfahren durch die folgenden Schritte **gekennzeichnet** ist: (i) Auftragen einer reduzierenden Zusammensetzung, die eine Kombination Disulfid/Thiol in einem Gewichtsverhältnis von Disulfid/Thiol im Bereich von 0,1 bis 1 enthält, auf die zu behandelnden Keratinfasern, wobei die Mittel, d.h. die Rollen, die erforderlich sind, um die Keratinfasern unter mechanische Spannung zu setzen, vor dem Auftragen oder während des Auftragens angebracht werden, (ii) Spülen der Keratinfasern, (iii) Entfernen der in Schritt (i) verwendeten Mittel, mit denen die Keratinfasern unter Spannung gesetzt wurden, aus den Keratinfasern durch Abnehmen der Rollen von den Keratinfasern zwischen der Reduktionsphase und der Fixierphase, (iv) leichte Oxidation der Keratinfasern, die darin besteht, eine oxidierende Zusammensetzung mit einer Einwirkzeit unter 2 min auf die Keratinfasern aufzubringen, und (v) gegebenenfalls erneutes Spülen der Keratinfasern.

2. Verfahren nach Anspruch 1, **dadurch gekennzeichnet, daß** das Gewichtsverhältnis Disulfid/Thiol im Bereich von 0,3 bis 0,8 liegt.

3. Verfahren nach Anspruch 1 oder 2, **dadurch gekennzeichnet, daß** die Kombination Disulfid/Thiol unter den folgenden Kombinationen ausgewählt ist: Dithiodiglykolsäure oder ihre Salze/Thioglykolsäure oder ihre Salze, Dithiodimilchsäure oder ihre Salze/Thiomilchsäure oder ihre Salze, Cystin/Cystein, Cystamin/Cysteamin, Mercaptopropionsäuredisulfid/Mercaptopropionsäure.

4. Verfahren nach einem der vorhergehenden Ansprüche, **dadurch gekennzeichnet, daß** das Thiol in der reduzierenden Zusammensetzung in einem Mengenanteil von 1 bis 20 Gew.-%, bezogen auf das Gesamtgewicht der Zusammensetzung, vorliegt.

5. Verfahren nach Anspruch 4, **dadurch gekennzeichnet, daß** der Mengenanteil im Bereich von 5 bis 15 % liegt.

6. Verfahren nach Anspruch 1, **dadurch gekennzeichnet, daß** die Einwirkungszeit unter 60 s liegt.

7. Verfahren nach einem der Ansprüche 1 bis 6, **dadurch gekennzeichnet, daß** die oxidierende Zusammensetzung eine Wasserstoffperoxidlösung enthält.

8. Verfahren nach Anspruch 7, **dadurch gekennzeichnet, daß** die Konzentration der Wasserstoffperoxidlösung in der oxidierenden Zusammensetzung im Bereich von 1 bis 9 Volumina liegt.

9. Verfahren nach Anspruch 8, **dadurch gekennzeichnet, daß** die Konzentration im Bereich von 1 bis 8 Volumina liegt.

10. Verfahren nach einem der Ansprüche 1 bis 9, **dadurch gekennzeichnet, daß** die oxidierende Zusammensetzung als Haarwaschmittel, Gel, Schaum, dickflüssige Lotion oder Creme vorliegt.
